# EUROPEAN PATENT SPECIFICATION

(11) **EP 2 745 849 B1**
(45) Date of publication and mention of the grant of the patent: **27.07.2022**
(21) Application number: 13197549.2
(22) Date of filing: 16.12.2013
(51) Int. Cl.: A61K 48/00, A61P 17/02, A61K 31/722, A61K 31/711, A61K 9/51, A61K 9/08, A61K 47/36, C12N 5/00

(54) **Polydeoxyribonucleotides composition and uses thereof**
Polydeoxyribonukleotidzusammensetzung und Verwendungen davon
Composition de polydeoxyribonucleotides et leurs utilisations

(30) Priority: 19.12.2012 IT TO20121107
(43) Date of publication of application: 25.06.2014
(73) Proprietor: ARCHIMEDES S.r.l., 35123 Padova (PD) (IT)
(72) Inventor: Ornaghi, Oscar Marco, 35031 Abano Terme (Padova) (IT); Binotto, Radames, 35031 Abano Terme (Padova) (IT); Binotto, Renzo, 35031 Abano Terme (Padova) (IT); Vallana, Valerio, 35031 Abano Terme (Padova) (IT)
(74) Representative: Susanetto, Carlo

(56) References cited:
- WO-A1-00/45825
- WO-A1-99/36090
- WO-A1-2009/039657
- WO-A2-03/092618
- KAM W. LEONG ET AL: NATURE MEDICINE, vol. 5, no. 4, 1 April 1999 (1999-04-01), pages 387-391, XP055078389, ISSN: 1078-8956, DOI: 10.1038/7385
- MUZZARELLI ET AL: "Chitins and chitosans for the repair of wounded skin, nerve, cartilage and bone", CARBOHYDRATE POLYMERS, APPLIED SCIENCE PUBLISHERS, LTD. BARKING, GB, vol. 76, no. 2, 17 March 2009 (2009-03-17) , pages 167-182, XP025913412, ISSN: 0144-8617, DOI: 10.1016/J.CARBPOL.2008.11.002 [retrieved on 2008-11-13]

## Description

### FIELD OF THE INVENTION

The present description concerns a polydeoxyribonucleotide (PDRN)-based composition able to stimulate cell proliferation.

### TECHNOLOGICAL BACKGROUND

Substances and compositions capable of stimulating cell proliferation are of great interest in the field of regenerative medicine.

Such compositions find application, for example, in the treatment of lacerations due to traumatic events or surgical wounds.

Several classes of substances are known that can promote the growth of active cells in tissue regeneration, such as hyaluronic acid, polylactic acid, nucleic acids, nucleic acid fractions including polydeoxyribonucleotides (PDRN).

In particular, PDRNs are used in plastic surgery due to their ability to stimulate fibroblast metabolism and the production of dermal matrix components.

PDRNs are also able to promote angiogenesis in the treatment of wounds and burns, to accelerate the healing of skin micro-lesions and the production of cytokines and growth factors.

The use is also known of compositions based on deoxyribonucleic acid (DNA) extracted for example from sturgeon gonad tissue and salified as a disodium salt, to obtain PDRN-Na.

In addition to finding application in diseases caused by a functional deficiency of immune processes, PDRN-Na has an immunomodulatory capacity that allows inflammation to be controlled, optimizing tissue re-Muzzarelli, Riccardo A. A. (2008), "Chitins and chitosans for the repair of wounded skin, nerve, cartilage and bone", Carbohydrate Polymers, 76, 167-182 reviews the use of chitins and chitosans in tissue regeneration.

Although substances capable of stimulating tissue regeneration are known, it is of considerable interest to identify new substances or compositions that can improve cellular proliferative capacity and be used in the very diverse applications of regenerative medicine such as for example in plastic surgery, in the treatment of postoperative complications, in the treatment of burns, of sores related to diabetic foot, of bedsores, of open wounds treated with radiotherapy that have impaired healing, of psoriatic manifestations.

### SUMMARY OF THE INVENTION

The object of the present description is to provide a composition capable of further improving the stimulation of cell proliferation by means of the synergistic effects exerted by its components.

In accordance with the invention, the above object is achieved by means of the solution recalled specifically in the annexed claims, which constitute an integral part of the present description.

One embodiment of the present description relates to a composition comprising polydeoxyribonucleotides (PDRN) and chitosan, the PDRN being employed in the form of salts.

The results given below show that the presence of chitosan in the composition comprising salified PDRN determines a considerable increase in cell proliferation compared to the use of compositions comprising PDRN, but without chitosan.

### DETAILED DESCRIPTION OF SOME EMBODIMENTS

In the following description, numerous specific details are given to provide a complete understanding of the embodiments. The embodiments may be practiced without one or more of the specific details, or with other methods, components, materials, etc. In other cases, well-known structures, materials, or operations are not shown or described in detail to avoid obscuring aspects of the embodiments.

Reference throughout the present description to "one embodiment" or "an embodiment" means that an aspect, structure, or characteristic described in relation to the particular embodiment is included in at least one embodiment. Therefore, the expressions "in one embodiment" or "in an embodiment" appearing in various places throughout the present description do not necessarily all refer to the same embodiment. Furthermore, the aspects, structures, or characteristics may be combined in any suitable way in one or more embodiments.

The headings given here are provided only for the purpose of convenience and do not interpret the scope or meaning of the embodiments.

While the exemplary embodiments described in detail below refer primarily to the use of the compositions described herein in relation to fibroblast proliferation, it will be understood that the scope of this description is not in any way limited to such envisaged use, since the compositions described herein may be used with any cell type such as, for example, keratinocytes, endothelial cells, osteoblasts.

The present description relates to a composition comprising polydeoxyribonucleotides (PDRN) and chitosan, where the PDRN are employed in the form of salt such as salt of sodium, iron, chromium, magnesium, manganese and others.

Chitosan is a deacetylated derivative of chitin, commonly extracted from the shells of crustaceans and from the cell wall of fungi.

The chitosan used in the composition object of the present description may have a molecular weight between 250 and 1 MDa, preferably equal to 1 MDa.

The composition comprises PDRN in the form of a salt in an amount that may be comprised between 20 and 2500 µg/ml, preferably equal to 80 µg/ml and chitosan in an amount that may be comprised between 1 and 10% w/v, preferably equal to 3% w/v.

Due to the synergy between the effects exerted by the PDRN and by chitosan, the composition described herein provides a considerable increase in cell proliferation, as shown below.

The composition can be obtained through a process that envisions the following steps:
- preparing a first solution containing PDRN in the form of a salt;
- preparing a second solution containing chitosan;
- mixing the first solution and the second solution to obtain the composition in liquid form.
- optionally leaving the composition to stand for a period comprised between 8 and 24 hours, preferably equal to 16 hours, to obtain a dispersion.

The first solution comprises polydeoxyribonucleotides in an amount comprised between 15 and 50 mg/ml, preferably equal to 25 mg/ml.

The second solution comprises chitosan in an amount comprised between 1 and 10% w/v, preferably equal to 6% w/v.

The composition obtained comprises PDRN in the form of a salt in an amount comprised between 20 and 2500 µg/ml, preferably equal to 80 µg/ml and chitosan in an amount comprised between 1 and 10% w/v, preferably equal to 3% w/v.

The physical form that the described composition assumes (liquid solution or dispersion) depends on the operating conditions adopted during the preparation (such as, for example, pH, temperature, time interval in which the composition is left to stand, etc.).

The composition may also be made for example in the form of a cream, ointment, gel, suspension or solution, the solution being suitable for administration by injection or spray, or as a lyophilized product for reconstitution before use with water or other suitable vehicles.

Such a composition may contain excipients and/or carriers suitable for the chosen route of administration such as preservatives, thickeners, antioxidants, emollients, moisturizers, natural or artificial fragrances.

The composition object of the present description may be used as a pharmaceutical composition whenever a cellular proliferative effect is required in regenerative medicine, for example in plastic surgery, and in the treatment of post-operative complications for the treatment of wounds, the treatment of burns, of diabetic foot sores, of bedsores, of open wounds treated with radiotherapy that have impaired healing, of the manifestations of psoriasis, in the treatment of ulcerous sores, for the detersion of open wounds, for stimulation of granulation, for stimulation of the dermal micro-environment for tissue regeneration.

Moreover, the composition object of the present description will find application also at the cosmetic level, for example for the treatment of scars, keloids, stretch marks, cellulite, wrinkles, and for recovery in general of the optimal characteristics of the epithelium.

Several preferred embodiments of the present invention will be described in detail below, referring to the use of PDRN in the form of a sodium salt (PDRN-Na) .

However, the scope of this description, is in no way limited to the use of PDRN-Na, as the operating conditions shown in the Examples that follow can be practiced in the same way to prepare compositions comprising PDRN in the form of other salts such as, for example, salts of sodium, iron, chromium, magnesium, manganese and others.

### Example 1. Effect of the PDRN-Na and chitosan composition on cell proliferation

PDRN in the form of sodium salt (Na-PDRN, Veritas Ltd.; molecular weight between 150 and 500 kDa, preferably equal to about 350 kDa; hyperchromicity >37%; DNA between 86% and 100%, RNA <8%; protein <0.7%, ratio of nitrogen/phosphorus between 1.3 and 1.8) was dissolved in 50 mM Tris, pH 7.5 to obtain a first solution at a concentration of 25 mg/ml.

Chitosan (1 MDa) was dissolved in water to obtain a second solution at 6% w/v.

Human fibroblasts (HFF-1, ATCC^{®} No: SCRC-1041^{™}) were used for the experiments described herein.

The monolayer of human fibroblasts was dissociated by incubation with trypsin and the cells thus obtained were placed in culture medium Dulbecco's Modified Eagle's Medium (DMEM; ATCC Catalog No. 30-2002) supplemented with fetal bovine serum (BSA), 10% v/v, penicillin and streptomycin, 1% v/v at a concentration of 100,000 cells/ml.

In a first set of experiments, the fibroblasts (100,000 cells/ml) were resuspended in culture medium (final volume 1 ml) supplemented with PDRN-Na (first solution at a dilution 1:312.15 so to obtain a final concentration of 80 µg/ml) and chitosan (second solution at a dilution to give a final concentration of 3% w/v).

In a second set of experiments, the fibroblasts were resuspended in culture medium (100,000 cells in a final volume of 1 ml) without the addition of other compounds (control experiment).

The cells were then seeded on 96-well plates (in a final volume of 100 µl) and cell proliferation was evaluated after 48 hours by means of the MTT assay.

The number of cells was calculated by extrapolation from a calibration curve prepared in parallel to the assay.

The data reported in Table 1 show that the composition containing PDRN-Na combined with chitosan determines a significant increase in the number of fibroblasts compared to the number of fibroblasts in culture medium only (control cells, CTR).

**Table 1**

| | **CTR** | **PDRN-Na +chitosan** |
|---|---|---|
| | 8,682.51 | 11,692.13 |
| | 8,832.99 | 10,638.77 |
| | 8,682.51 | 10,789.25 |
| **MEAN** | 8,733.00 | 11,040.00 |
| **STD ERROR** | 50.16 | 328.90 |

Each experimental condition was repeated in triplicate. The data shown represent the mean and standard error of the number of fibroblasts analyzed and significance was calculated with a One-way ANOVA test and the Newman-Keuls post-hoc test (p<0.05).

### Example 2. Effect of PDRN-Na and PDRN-Na + chitosan compositions on cell proliferation

PDRN-Na were dissolved in 50 mM Tris, pH 7.5 in a first solution at a concentration of 25 mg/ml.

Chitosan (1 MDa) was prepared in a second solution at 6% w/v in water.

In a first set of experiments, an aliquot of the second solution containing chitosan was added to 1 ml of culture medium in order to obtain a final chitosan concentration of 3% w/v.

After the addition of chitosan, the culture medium was allowed to stand for 16 hours.

Human fibroblasts (100,000 cells/ml) were resuspended in culture medium supplemented with chitosan.

In a second set of experiments, 1 ml of culture medium was supplemented with:
- an aliquot of the first solution containing PDRN-Na (to obtain a final concentration of 80 µg/ml) and
- an aliquot of the second solution containing chitosan (to obtain a final concentration of 3% w/v).

Human fibroblasts (100,000 cells/ml) were resuspended in culture medium supplemented with PDRN-Na and chitosan, as described above, 16 hours after it was prepared.

In a third set of experiments, human fibroblasts were resuspended in culture medium (100,000 cells in a final volume of 1 ml) without the addition of other compounds as control experiments.

Each experimental condition was evaluated in triplicate.

The data reported in Table 2 show that the composition containing chitosan and PDRN-Na determines a significant increase (p<0.001) of the proliferation of fibroblasts compared to a control composition and compared to a composition containing only chitosan.

**Table 2**

| | **CTR** | **chitosan** | **PDRN-Na +chitosan** |
|---|---|---|---|
| | 12,143.60 | 28,124.90 | 89,386.55 |
| | 9,923.97 | 25,461.35 | 144,877.20 |
| | 9,923.75 | 29,456.68 | 132,003.40 |
| **MEAN** | 10,664.00 | 27,681.00 | 122,089.00 |
| **STD ERROR** | 739.90 | 1,175.00 | 16,768.00 |

### Example 3. Effect of PDRN-Na and PDRN-Na and chitosan compositions deposited on plastic supports.

The effect of the composition comprising PDRN-Na and chitosan on the proliferation of human fibroblasts was assessed following their deposition on a plastic support onto which the cells were subsequently seeded (16 hours later).

The following solution were prepared:
- the first solution of PDRN-Na dissolved in 50 mM Tris, pH 7.5 at a concentration of 25 mg/ml.
- the second solution of chitosan 6% w/v in water.

The solutions were mixed to obtain a composition containing PDRN-Na at a final concentration of 80 µg/ml and chitosan at a final concentration of 3% w/v.

One hundred µl of the above solution (PDRN-Na and chitosan) were deposited on the bottom of the wells of a plastic 96-well plate.

One hundred µl of a solution containing Na-PDRN without chitosan were deposited on the bottom of other wells.

One hundred µl of a solution lacking the above components (essentially constituted by culture medium) were deposited in the control wells.

The solution deposited in each well was allowed to stand for 16 hours at room temperature in a continuous laminar flow hood.

After this time interval, human fibroblasts (100,000 cells/ml) were seeded into the wells and incubated for 48 hours.

As reported in Table 3, the PDRN-Na and chitosan composition determines a significant increase in the number of fibroblasts equal to 15-fold compared to the number of fibroblasts seeded in the control wells (p<0.001) and equal to about 13-fold compared to the number of fibroblasts seeded on the wells treated with only PDRN-Na (p<0.001).

**Table 3**

| | **CTR** | **PDRN-Na** | **PDRN-Na +chitosan** |
|---|---|---|---|
| | 7,270.01 | 9,666.08 | 103,511.90 |
| | 7,270.01 | 8,867.39 | 124,677.10 |
| | 10,464.77 | 10,464.77 | 139,852.20 |
| **MEAN** | 8,335.00 | 9,666.00 | 122,680.00 |
| **STD ERROR** | 1,065.00 | 461.10 | 10,538.00 |

### Example 3a. Effect of PDRN-Na and PDRN-Na and chitosan compositions deposited on plastic supports.

The experimental conditions described in Example 3 were reproduced under the same experimental conditions and the results are reported in Table 4.

In confirmation of what was observed and described for Example 3, the PDRN-Na and chitosan composition deposited on a support and allowed to stand for 16 hours determined a significant increase in cell proliferation compared to control cells (p<0.001) and compared to a composition containing PDRN-Na but lacking chitosan, as reported in Table 4.

**Table 4**

| | **CTR** | **PDRN-Na** | **PDRN-Na +chitosan** |
|---|---|---|---|
| | 8,093.22 | 8,785.73 | 97,678.65 |
| | 5,723.25 | 11,552.21 | 95,412.16 |
| | 4,733.43 | 13,041.22 | 90,879.19 |
| **MEAN** | 6,183.00 | 11,126.00 | 94,657.00 |
| **STD ERROR** | 996.80 | 1,247.00 | 1,999.00 |

Also in these tests, the experimental analysis was repeated in triplicate. The data shown represent the mean and standard error of the number of fibroblasts analyzed and significance was calculated using a One-way ANOVA test and the Newman-Keuls post-hoc test (GraphPad Prism).

Naturally, while the principle of the invention remains constant, the structural details and the embodiments may vary widely with respect to what has been described and illustrated by way of example only, without departing from the scope of the present invention as specified in the annexed claims.

## Claims

1. A composition comprising polydeoxyribonucleotides (PDRN) and chitosan, wherein said polydeoxyribonucleotides are present in the form of a salt, and have:
- molecular weight between 150 and 500 kDa;
- hyperchromicity >37%;
- DNA between 86% and 100%;
- RNA <8%;
- protein <0.7% and
- ratio of nitrogen/phosphorus between 1.3 and 1.8.

2. The composition according to claim 1, wherein said salt is selected from among: sodium, iron, chromium, magnesium, manganese salt.

3. The composition according to any one of the preceding claims, wherein said polydeoxyribonucleotides are present in an amount comprised between 20 and 2500 µg/ml, preferably equal to 80 µg/ml.

4. The composition according to any one of the preceding claims, wherein said chitosan is present in an amount comprised between 1 and 10% w/v, preferably equal to 3% w/v.

5. The composition according to any one of the preceding claims, wherein said polydeoxyribonucleotides has a molecular weight of about 350 kDa.

6. The composition according to any one of the preceding claims, wherein said chitosan has a molecular weight of between 250 kDa and 1 MDa, preferably equal to about 1 MDa.

7. A process for the preparation of a composition comprising polydeoxyribonucleotides (PDRN) and chitosan, comprising:
- preparing a first solution containing polydeoxyribonucleotides in the form of a salt in an amount between 15 and 50 mg/ml, preferably equal to 25 mg/ml;
- preparing a second solution containing chitosan in an amount between 1 and 10% w/v, preferably equal to 6% w/v;
- mixing the first solution and the second solution to obtain a composition in liquid form comprising PDRN in the form of a salt in an amount comprised between 20 and 2500 µg/ml and chitosan in an amount comprised between 1 and 10% w/v.

8. The process according to claim 7, wherein the method further comprises the step of leaving the composition to stand for a period comprised between 8 and 24 hours, preferably equal to 16 hours, obtaining a composition in dispersion form.

9. The composition according to any one of the claims 1 to 6, for use in the treatment of wounds, burns, diabetic foot sores, bedsores, open wounds treated with radiotherapy, psoriasis manifestations, ulcerative sores.

10. The composition according to any one of the claims 1 to 6 for use in the detersion of wounds.

11. A cosmetic product comprising a composition according to any one of claims 1 to 6.

## Patentansprüche

1. Zusammensetzung, die Polydesoxyribonukleotide (PDRN) und Chitosan aufweist, wobei die Polydesoxyribonukleotide in Form eines Salzes vorliegen, und
- Molekulargewicht zwischen 150 und 500 kDa;
- Hyperchromizität >37 %;
- DNA zwischen 86 % und 100 %;
- RNA < 8 %;
- Protein < 0,7 % und
- Stickstoff/Phosphor-Verhältnis zwischen 1,3 und 1,8 haben.

2. Zusammensetzung nach Anspruch 1, wobei das Salz ausgewählt ist aus: Natrium, Eisen, Chrom, Magnesium, Mangansalz.

3. Zusammensetzung nach einem der vorhergehenden Ansprüche, wobei die Polydesoxyribonukleotide in einer Menge zwischen 20 und 2500 µg/ml vorhanden sind, vorzugsweise gleich 80 µg/ml.

4. Zusammensetzung nach einem der vorhergehenden Ansprüche, wobei das Chitosan in einer Menge zwischen 1 und 10 % G/V vorhanden ist, vorzugsweise gleich 3 % G/V.

5. Zusammensetzung nach einem der vorhergehenden Ansprüche, wobei die Polydesoxyribonukleotide ein Molekulargewicht von etwa 350 kDa haben.

6. Zusammensetzung nach einem der vorhergehenden Ansprüche, wobei das Chitosan ein molekulares Gewicht zwischen 250 kDa und 1 MDa hat, vorzugsweise gleich etwa 1 MDa.

7. Verfahren zur Herstellung einer Zusammensetzung, die Polydesoxyribonukleotide (PDRN) und Chitosan aufweist, umfassend:
- Herstellen einer ersten Lösung enthaltend Polydesoxyribonukleotide in Form eines Salzes in einer Menge zwischen 15 und 50 mg/ml, vorzugsweise gleich 25 mg/ml;
- Herstellen einer zweiten Lösung, die Chitosan in einer Menge zwischen 1 und 10 % G/V enthält, vorzugsweise gleich 6 % G/V;
- Mischen der ersten Lösung und der zweiten Lösung, um eine Zusammensetzung in flüssiger Form zu erhalten, die PDRN in Form eines Salzes in einer Menge zwischen 20 und 2500 µg/ml und Chitosan in einer Menge zwischen 1 und 10 % G/V aufweist.

8. Verfahren nach Anspruch 7, wobei das Verfahren ferner den Schritt des Stehenlassens der Zusammensetzung für einen Zeitraum zwischen 8 und 24 Stunden, vorzugsweise gleich 16 Stunden, umfasst, wodurch eine Zusammensetzung in Dispersionsform erhalten wird.

9. Zusammensetzung nach einem der Ansprüche 1 bis 6 zur Verwendung bei der Behandlung von Wunden, Verbrennungen, diabetischen Fußgeschwüren, Dekubitus, mit Strahlentherapie behandelten offenen Wunden, Psoriasis-Manifestationen, ulzerativen Wunden.

10. Zusammensetzung nach einem der Ansprüche 1 bis 6 zur Verwendung bei der Reinigung von Wunden.

11. Kosmetisches Produkt, das eine Zusammensetzung nach einem der Ansprüche 1 bis 6 aufweist.

## Revendications

1. Composition comprenant des polydésoxyribonucléotides (PDRN) et du chitosane, où lesdits polydésoxyribonucléotides sont présents sous forme de sel, et ont:
- un poids moléculaire entre 150 et 500 kDa ;
- une hyperchromicité >37 % ;
- de l'ADN entre 86 % et 100 % ;
- de l'ARN <8 % ;
- des protéines <0,7 % et
- un rapport azote/phosphore compris entre 1,3 et 1,8.

2. Composition selon la revendication 1, où ledit sel est choisi parmi :
un sel de sodium, de fer, de chrome, de magnésium, de manganèse.

3. Composition selon l'une quelconque des revendications précédentes, où lesdits polydésoxyribonucléotides sont présents en une quantité comprise entre 20 et 2500 µg/ml, de préférence égale à 80 µg/ml,

4. Composition selon l'une quelconque des revendications précédentes, où ledit chitosane est présent en une quantité comprise entre 1 et 10 % p/v, de préférence égale à 3 % p/v.

5. Composition selon l'une quelconque des revendications précédentes, où lesdits polydésoxyribonucléotides ont un poids moléculaire d'environ 350 kDa.

6. Composition selon l'une quelconque des revendications précédentes, où ledit chitosane a un poids moléculaire entre 250 kDa et 1 MDa, de préférence égal à environ 1 MDa.

7. Procédé de préparation d'une composition comprenant des polydésoxyribonucléotides (PDRN) et du chitosane, comprenant :
- la préparation d'une première solution contenant des polydésoxyribonucléotides sous forme d'un sel en une quantité entre 15 et 50 mg/ml, de préférence égale à 25 mg/ml ;
- la préparation d'une seconde solution contenant du chitosane en une quantité entre 1 et 10 % p/v, de préférence égale à 6 % p/v ;
- le mélange de la première solution et la seconde solution pour obtenir une composition sous forme liquide comprenant des PDRN sous la forme d'un sel en une quantité comprise entre 20 et 2500 µg/ml et du chitosane en une quantité comprise entre 1 et 10 % p/v.

8. Procédé selon la revendication 7, où le procédé comprend en outre l'étape de laisser reposer la composition pendant une durée comprise entre 8 et 24 heures, de préférence égale à 16 heures, obtenant une composition sous forme de dispersion.

9. Composition selon l'une quelconque des revendications 1 à 6, destinée à être utilisée dans le traitement des plaies, des brûlures, des plaies du pied diabétiques, des escarres, des plaies ouvertes traitées par radiothérapie, des manifestations psoriasiques, des plaies ulcéreuses.

10. Composition selon l'une quelconque des revendications 1 à 6 destinée à être utilisée dans la détersion des plaies.

11. Produit cosmétique comprenant une composition selon l'une quelconque des revendications 1 à 6.
